(19) Europäisches Patentamt
European Patent Office
Office européen des brevets

(11) **EP 1 496 918 B1**

(12) **EUROPEAN PATENT SPECIFICATION**

(45) Date of publication and mention
of the grant of the patent:
**08.02.2023 Bulletin 2023/06**

(21) Application number: **02722968.1**

(22) Date of filing: **10.04.2002**

(51) International Patent Classification (IPC):
**A61K 33/36** (1974.07)     **A61P 35/00** (2000.01)

(52) Cooperative Patent Classification (CPC):
(C-Sets available)
**A61K 33/36; A61K 31/00; A61K 33/34;
A61K 45/06; A61P 35/00**              (Cont.)

(86) International application number:
**PCT/NL2002/000231**

(87) International publication number:
**WO 2003/086424 (23.10.2003 Gazette 2003/43)**

(54) **USE OF SODIUM META-ARSENITE FOR THE TREATMENT OF TUMOURS**

VERWENDUNG VON NATRIUM META-ARSENIT ZUR BEHANDLUNG VON TUMOREN

UTILISATION DE META-ARSENITE DE SODIUM POUR LE TRAITEMENT DES TUMEURS

(84) Designated Contracting States:
**AT BE CH CY DE DK ES FI FR GB GR IE IT LI LU
MC NL PT SE TR**
Designated Extension States:
**LT**

(43) Date of publication of application:
**19.01.2005 Bulletin 2005/03**

(60) Divisional application:
**08168243.7 / 2 042 182**

(73) Proprietor: **Komipharm International Co., Ltd.
Jungwang-Dong
Shihung City
Kyonggi do (KR)**

(72) Inventors:
• **LEE, Sang Bong
Anyang City,
Kyonggi-Do (KR)**
• **YANG, Yong Jin
Seongnam-City,
Kyonggi-Do (KR)**

(74) Representative: **Sharples, Andrew John et al
EIP
Fairfax House
15 Fulwood Place
London WC1V 6HU (GB)**

(56) References cited:
**EP-A- 0 804 928     WO-A-99/18798**

• **KIM H-S ET AL: "Intracellular Glutathione Level
Modulates the Induction of Apoptosis by
DELTA-Prostaglandin J2" PROSTAGLANDINS,
BUTTERWORTH, STONEHAM, MA, US, vol. 51,
no. 6, 1 June 1996 (1996-06-01), pages 413-425,
XP004032382 ISSN: 0090-6980**
• **WAXMAN, S., ANDERSON, K.C.: "History of the
Development of Arsenic Derivatives in Cancer
Therapy" THE ONCOLOGIST, vol. 6, no. 2, 2001,
pages 3-10, XP001087583**

Remarks:
The file contains technical information submitted after
the application was filed and not included in this
specification

EP 1 496 918 B1

(52) Cooperative Patent Classification (CPC): (Cont.)

C-Sets
**A61K 31/00, A61K 2300/00;**
**A61K 33/34, A61K 2300/00**

**Description**

The present invention relates to the use of meta-arsenite for the manufacture of a pharmaceutical composition for the treatment of a solid malignancy according to the claims.

Pharmaceutical compositions containing arsenic are known for cancer therapy. For example, the review by Waxman S. et al (The Oncologist 6(suppl. 2), pp. 3-10 (2001)) describes arsenic disulfide, arsenic trisulfide and arsenic trioxide the use of said composition comprising a salt of meta-arsenite ($AsO_2^-$) and a pharmaceutically acceptable auxiliary.

Due to their inherent toxicity, and the advent of good alternatives, interest in arsenic compounds has remained low.

The object of the present invention is to provide a pharmaceutical composition suitable for use in cancer therapy, which allows for the treatment of solid tumours. The pharmaceutical composition may be used for treatment of such solid tumours for which currently no treatment exists, or as an alternative or supplementary treatment for such solid tumours.

To this end, the pharmaceutical composition according to the present invention is characterized in that it is a pharmaceutical composition for the treatment of a solid malignancy chosen from colon tumour, gastric tumour, mammary tumour, ovarian tumour, prostate tumour, and renal tumour, said composition comprising a salt of meta-arsenite ($AsO_2^-$) and a pharmaceutically acceptable auxiliary.

It has been found that the specified types of tumour are surprisingly sensitive to the meta-arsenite salt. In the salt the counter-ion of meta-aresenite may be any pharmaceutically acceptable counter-ion.

In the article by Waxman, mention is made of an ar- . ticle by Tarnowski G.S. et al (Cancer Research, 26(2), pp. 181-206 (1966)) where 8 tumour types were investigated with 14 different anti-tumour chemicals. Potassium arsenite affected only the growth of Ehrlich ascites tumour. Regarding this tumour type it is remarked that it is more sensitive in the ascites than in the sold form. This emphasizes the surprising finding of the present invention.

According to a preferred embodiment, the salt is an alkaline or earth alkaline metal salt. According to a more preferred embodiment, the alkaline metal salt is a potassium or sodium salt.

Such salts are readily soluble and are readily available for exerting their anti-tumour effect.

The invention relates to the use of a salt of meta-arsenite ($AsO_2^-$) for the manufacture of a pharmaceutical composition for the treatment of a solid malignancy chosen from colon tumour, gastric tumour, mammary tumour, ovarian tumour, prostate tumour, and renal tumour.

It was found that solid malignancy belonging to the group consisting of colon tumour, mammary tumour, prostate tumour, and renal tumour were particularly sensitive.

The present invention will now be elucidated with reference to the following example.

EXAMPLE

Various human tumor cells were grown at 37°C in a humidified atmosphere (95% air, 5% $CO_2$) in monolayer cultures in RPMI 1640 medium with phenol red (Life Technologies, Karlsruhe, Germany) supplemented with 10% fetal calf serum. Cells were trypsinized and maintained weekly.

**Cytotoxicity assay**

A modified propidium iodide assay (based on W.A. Dengler et. al, Anti-Cancer Drugs, 6, pp. 522-532 (1995)) was used to examine the antiproliferative activity of the study compounds. Briefly, cells will be harvested from exponential phase cultures growing in RPMI 1640 medium supplemented with 10% fetal calf serum by trypsination, counted and plated in 96 well flat-bottomed microtiter plates (140 $\mu$l cell suspension, a $\times$ $10^4$ cells/ml). After a 24h recovery, to allow cells to resume exponential growth, 10 $\mu$l culture medium (6 control wells. per plate) or culture medium containing the test drug were added to the wells. Each drug concentration was plated in triplicate. After 4 days of incubation culture medium was replaced by an aqueous propidium iodide solution (6 $\mu$g/ml). Microtiter plates were kept at -18°C for 24 h, resulting in a total cell kill. After thawing of the plates, fluorescence was measured using a Millipore Cytofluor 2350-microplate reader (excitation 530 nm, emission 620 nm) in order to quantify the total cell number. The assay included untreated and positive controls (5-FU and vindesine).

Growth inhibition is expressed as Treated/Control x 100 (or T/C%) . $IC_{50}$ and $IC_{70}$ values were determined by plotting compound concentration versus cell number. Mean $IC_{50}$ and $IC_{70}$ values were calculated according to the formula:

$$\text{Mean } IC_{50,70} = \frac{\sum_{x=1}^{n} \log (IC_{50,70})_x}{10^n}$$

With x = specific tumor cell line and n = total number of cell lines studied. If $IC_{50}$ or $IC_{70}$ could not be determined within the examined dose range, the lowest or highest concentration studied was used for the calculation.

Assays were considered valid only if the positive control (5-FU) induced a tumor growth inhibition of T/C <30%' and if vehicle treated control cells had a fluorescence intensity >500 units.

## Results

The results have been summarized in Table I, which shows that in particular tumour cell lines of the type gastric tumour, ovarian tumour, and in particular prostate tumour, mammary tumour, renal and colon tumour were sensitive to the meta-arsenite compound. In comparison, promyelocytic leukaemia, which is known to respond to arsenic trioxide, showed an $IC_{70}$ value of 6.82 $\mu$g/ml. Hence, the tumour cells to which the present invention relates are about 2 to 20 times more sensitive to the meta-arsenite compound according to the present invention.

TABLE I

| Tumour | cell line | $IC_{70}$ ($\mu$g/ml) |
|---|---|---|
| Colon | DLD1 | 0.48 |
| Gastric | GXF251L | 3.08 |
| Mammary | MXAF401NL | 0.32 |
| Ovarian | OVCAR3 | 3.02 |
| Prostate | PC3 | 0.85 |
| Renal | RXF986L | 0.63 |

## Claims

1.  Use of sodium meta-arsenite (AsO.sub.2.sup.-) for manufacture of a pharmaceutical composition for the treatment of a solid malignancy chosen from the group consisting of colon tumour, gastric tumour, mammary tumour, ovarian tumour, prostate tumour and renal tumour.

2.  Use according to claim 1, wherein said solid malignancy is a colon tumour.

3.  Use according to claim 1, wherein said solid malignancy is a gastric tumour.

4.  Use according to claim 1, wherein said solid malignancy is a mammary tumour.

5.  Use according to claim 1, wherein said solid malignancy is a ovarian tumour.

6.  Use according to claim 1, wherein said solid malignancy is a prostate tumour.

7.  Use according to claim 1, wherein said solid malignancy is a renal tumour.

## Patentansprüche

1.  Verwendung von Natriummetaarsenit (AsO$_2^-$) zur Herstellung einer pharmazeutischen Zusammensetzung zur Behandlung eines festen bösartigen Tumors, ausgewählt aus der Gruppe, bestehend aus Dickdarmtumor, Magentumor, Brusttumor, Eierstocktumor, Prostatatumor und Nierentumor.

2.  Verwendung gemäß Anspruch 1, wobei der feste bösartige Tumor ein Dickdarmtumor ist.

3.  Verwendung gemäß Anspruch 1, wobei der feste bösartige Tumor ein Magentumor ist.

4.  Verwendung gemäß Anspruch 1, wobei der feste bösartige Tumor ein Brusttumor ist.

5.  Verwendung gemäß Anspruch 1, wobei der feste bösartige Tumor ein Eierstocktumor ist.

6.  Verwendung gemäß Anspruch 1, wobei der feste bösartige Tumor ein Prostatatumor ist.

7.  Verwendung gemäß Anspruch 1, wobei der feste bösartige Tumor ein Nierentumor ist.

## Revendications

1.  Utilisation de meta-arsénite de sodium (AsO.sub.2.sup.-) pour la fabrication d'une composition pharmaceutique pour le traitement d'une tumeur maligne solide du groupe formé des tumeurs du côlon, des tumeurs gastriques, des tumeurs mammaires, des tumeurs ovariennes, des tumeurs de la prostate et des tumeurs rénales.

2.  Utilisation selon la revendication 1, dans laquelle ladite tumeur maligne solide est une tumeur du côlon.

3.  Utilisation selon la revendication 1, dans laquelle ladite tumeur maligne solide est une tumeur gastrique.

4.  Utilisation selon la revendication 1, dans laquelle ladite tumeur maligne solide est une tumeur mammaire.

5.  Utilisation selon la revendication 1, dans laquelle ladite tumeur maligne solide est une tumeur ovarienne.

6.  Utilisation selon la revendication 1, dans laquelle ladite tumeur maligne solide est une tumeur de la prostate.

7.  Utilisation selon la revendication 1, dans laquelle ladite tumeur maligne solide est une tumeur rénale.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WAXMAN S. et al.** *The Oncologist,* 2001, vol. 6 (2), 3-10
- **TARNOWSKI G.S. et al.** *Cancer Research,* 1966, vol. 26 (2), 181-206
- **DENGLER.** *Anti-Cancer Drugs,* 1995, vol. 6, 522-532